# EUROPÄISCHE PATENTSCHRIFT

(11) **EP 4 288 822 B1**
(45) Veröffentlichungstag und Bekanntmachung des Hinweises auf die Patenterteilung: **14.10.2026**
(21) Anmeldenummer: 22703387.5
(22) Anmeldetag: 03.02.2022
(51) Int. Cl.: G02B 23/24, A61B 1/05, A61B 1/00, A61B 1/06

(54) **ENDOSKOPISCHES INSTRUMENT**
LENS FOR AN ENDOSCOPIC INSTRUMENT
LENTILLE POUR INSTRUMENT ENDOSCOPIQUE

(30) Priorität: 05.02.2021 EP 21155375
(43) Veröffentlichungstag der Anmeldung: 13.12.2023
(73) Patentinhaber: Richard Wolf GmbH, 75438 Knittlingen (DE)
(72) Erfinder: PFITZENMEIER, Bernd, 75438 Knittlingen (DE); FRANK, Alexander, 75438 Knittlingen (DE)
(74) Vertreter: Patentanwälte Hemmer Lindfeld Frese Partnerschaft mbB
(86) Internationale Anmeldenummer: PCT/EP2022/052555
(87) Internationale Veröffentlichungsnummer: WO 2022/167512

(56) Entgegenhaltungen:
- WO-A1-2015/054583
- WO-A1-2020/152782
- US-A1- 2019 090 720
- US-A1- 2020 271 957

## Beschreibung

Die Offenbarung betrifft ein endoskopisches Instrument sowie ein Verfahren zum Herstellen endoskopischer Instrumente.

Objektive für Bildsensoren eines endoskopischen Instruments weisen zur Minimierung der radialen Ausdehnung eines Bildgebungskanals in dem Schaft des Instruments üblicherweise einen sehr geringen Durchmesser von 1 mm oder weniger auf. Es ist bekannt, Kameramodule mit einer Bildsensoreinheit und einem darauf angeordneten Objektiv zur Integration in ein solches Instrument einzusetzen. Ein allgemein bekanntes Verfahren zur Herstellung von miniaturisierten Objektiven basiert auf dem Prinzip von sogenannten Wafer-Level-Optics (WLO). Dabei werden mehrere Substratschichten mit vorbestimmten optischen Eigenschaften miteinander verbunden und bilden ein Wafer-Paket, aus dem einzelne Objektive herausgeschnitten werden. Für den Trennprozess wird sich oftmals an der Größe und Form des Bildsensors orientiert. Dieser ist üblicherweise quadratisch, sodass das Objektiv einen korrespondierenden Querschnitt erhält. Soll nun ein solches bekanntes WLO-Objektiv bei einem endoskopischen Instrument in einem Bildgebungskanal mit weniger als 1 mm Durchmesser mit einer ausreichend großen optisch wirksamen Fläche zum Einsatz kommen, so muss es zunächst überdimensioniert sein und anschließend umfangseitig auf die Größe des Bildgebungskanal abgeschliffen bzw. abgerundet werden. Aufgrund der geringen Abmessungen des Objektivs ist das Abschleifen bzw. Abrunden allerdings aufwändig, da ein Schleifvorgang nur bei exakter Zentrierung zu einem präzisen Bauteil führt. Dies führt zu signifikanten Zusatzkosten und in der Konsequenz zu einem entsprechend hohen Stückpreis.

Aus der US 2019/090720 A1 ist ein Herstellungsverfahren einer optischen Einheit für ein Endoskop bekannt. Die WO 2020/152782 A1 beschreibt ebenfalls ein Herstellungsverfahren eines endoskopischen Bildgebungsgeräts.

Die Aufgabe der Erfindung liegt darin, ein endoskopisches Instrument mit einem Objektiv bereitzustellen, wobei das Objektiv in radialer Richtung besonders kompakt ist, dennoch eine größtmögliche optisch wirksame Fläche aufweist und gleichzeitig kostengünstig herstellbar ist.

Die Aufgabe wird durch den Gegenstand der Ansprüche 1 und 13 gelöst.

Gemäß einem ersten Aspekt der vorliegenden Offenbarung wird ein endoskopisches Instrument mit einem rohrförmigen Schaft bereitgestellt, an dessen distaler Spitze ein Objektiv angeordnet ist, wobei das Objektiv eine aus einem mehrschichtigen Wafer-Paket herausgetrennte Anordnung miteinander verbundener Objektivglieder aufweist, die jeweils vorgegebene optische Eigenschaften aufweisen und entlang einer optischen Achse aufeinander folgen. Gemäß der Erfindung ist vorgesehen, dass das Objektiv einen senkrecht zu der Bildachse polygonalen und zumindest sechseckigen Querschnitt aufweist. Dabei weist die distale Spitze einen Bildgebungskanal auf, wobei das Objektiv formschlüssig, reibschlüssig und/oder stoffschlüssig in dem Bildgebungskanal eingesetzt ist. Der Bildgebungskanal weist dabei einen ersten distalen Bildgebungskanalabschnitt auf, in dem das Objektiv eingesetzt ist, und einen proximal vom ersten Bildgebungskanalabschnitt angeordneten zweiten Bildgebungskanalabschnitt, der einen größeren Innendurchmesser aufweist als der erste Bildgebungskanalabschnitt, wobei eine mit dem Objektiv stoffschlüssig verbundene Bildsensoreinheit im zweiten Bildgebungskanalabschnitt angeordnet ist, wobei die Bildsensoreinheit größere laterale Abmessungen aufweist als das Objektiv.

Das Objektiv wird folglich ebenso mittels der Wafer-Level-Optics (WLO) Bauweise hergestellt. Das mehrschichtige Wafer-Paket umfasst mehrere Schichten aus einem optischen Material. Die erforderlichen Schichten können nach unterschiedlichen Verfahren gefertigt und unterschiedlich ausgebildet werden. Es ist nicht zwangsläufig notwendig, dass alle Schichten aus demselben Material bestehen. Beispielhaft könnte eine Schicht des Wafer-Pakets, das Linsen bereitstellt, aus einem UV-härtendem Polymer ausgebildet sein. Als Träger oder Basis dieser Schicht könnte wiederum ein Glassubstrat angeordnet sein. Je nach Aufgabe der betreffenden Schicht können auch andere Materialien ausgewählt werden. Dabei ist lediglich zu beachten, dass die gewünschten optischen Eigenschaften realisiert werden können und dass das resultierende Wafer-Paket auf die gewünschte Weise geschnitten werden kann.

Die einzelnen Schichten werden entlang der späteren Bildachse aufeinander gestapelt und miteinander verbunden. Die Verbindung kann beispielsweise durch einen Klebstoff, eine anodische Verbindung oder andere stoffschlüssige Verbindungsverfahren erreicht werden. Das Wafer-Paket erlaubt es, eine große Anzahl von Objektiven gleichzeitig zu fertigen, indem schichtweise Anordnungen mit Objektivgliedern hergestellt und anschließend aus dem Wafer-Paket ausgeschnitten werden.

Gemäß der Erfindung ist vorgesehen, dass die Anordnung einen polygonalen und zumindest sechseckigen Querschnitt aufweist. Die im Stand der Technik bekannte WLO-Bauweise der Objektive kann zu viereckigen und insbesondere quadratischen Querschnitten führen, welcher zu sehr großen Abmessungen im Verhältnis zur aktiven optischen Fläche führt. Durch den zumindest sechseckigen Querschnitt kann jedoch eine Kontur realisiert werden, die einem Kreis deutlich näher kommt als ein Quadrat. Nacharbeiten zur Anpassung des Querschnitts sind demgemäß nicht erforderlich. Zusätzlich zum Bildgebungskanal wird keine eigene Einfassung des Objektivs benötigt, da durch die dem Kreis näher kommende Kontur eine Einpassung direkt in den Bildgebungskanal möglich ist. Die Ausführung als Polygon erfordert lediglich einen Mehraufwand beim Schneiden des Wafers gegenüber quadratischen WLO-Objektiven. Durch vorteilhafte Anordnung der Objektivglieder auf den entsprechenden Schichten des Wafer-Pakets kann der Verschnitt jedoch relativ niedrig gehalten werden. Bei der Verwendung eines sechseckigen und insbesondere hexagonalen Querschnitts könnte es ausreichen, das Wafer-Paket mit Abfolgen paralleler Schnitte in drei Richtungen zu schneiden. Die optisch wirksame Fläche kann gegenüber einem in einen Bildgebungskanal eingepassten quadratischen Objektiv deutlich vergrößert werden.

In einer besonders bevorzugten Ausführungsform ist der Querschnitt gleichseitig polygonal und bevorzugt hexagonal oder oktogonal. Durch die gleichseitige Anordnung werden die Arbeiten zum Heraustrennen des Wafer-Pakets weiter reduziert. Insbesondere bei der hexagonalen Form kann der Verschnitt deutlich reduziert werden, wenn die Objektivglieder in den einzelnen Schichten des Wafer-Pakets beispielsweise spaltenweise um eine halbe Höhe des betreffenden Querschnitts zueinander versetzt sind.

Weiterhin ist es vorteilhaft, wenn die Objektivglieder ein erstes Endplättchen und ein zweites Endplättchen aufweisen, die die Anordnung an einander entgegensetzten stirnseitigen Enden begrenzen. Die Endplättchen könnten beispielsweise aus Glas, Quarzglas oder einem Polymer, etwa einem UV-härtenden Polymer, ausgebildet sein und begrenzen das Objektiv stirnseitig. Eine Linse oder ein anderes lichtbündelndes Objektivglied kann durch die beiden Endplättchen axial eingefasst und geschützt werden. Durch die Endplättchen werden beidseitig plane Abschlussflächen mit einer gewünschten Abriebfestigkeit für das distale Ende und einer geeigneten optischen Einkopplungsfläche für einen Bildsensor am proximalen Ende bereitgestellt.

Bevorzugt weisen die Objektivgliederzumindest eine Linse auf. Die Linse als wesentliches Bauteil zur optischen Abbildung kann an die jeweilige Ausführungsform des endoskopischen Instruments angepasst werden. Es ist denkbar, dass die Linse aus einem Kunststoff, etwa einem UV-härtenden Polymer, hergestellt ist. Zur Herstellung könnte sich ein Lithographie-Verfahren oder dergleichen auf einem Glassubstrat als Träger anbieten.

Es ist vorteilhaft, wenn die Linse eine asphärisch geformte Fläche aufweist, die von einem flachen Rand umgeben ist, wobei ein Distanzglied auf dem Rand angeordnet ist und sich entlang der optischen Achse über die sphärische Fläche hinaus erstreckt. Das Distanzglied schützt die Linse und kann diese axial von den angrenzenden Objektivgliedern separieren. Es ist denkbar, dass zwischen der Linse und dem Distanzglied ein zur Vermeidung von Kondensation angepasstes Fluid angeordnet bzw. eingeschlossen ist. Durch das Distanzglied ist die Linse auch ohne umfangsseitige Einfassung in radialer Richtung geschützt.

Die Linse könnte auf einer ebenen und durchgehenden Fläche eines Glassubstrats angeordnet sein. Das Glassubstrat könnte eine Basis zum Aufbau eines Linsenkörpers aus einem Polymer sein. Durch den Aufbau der Linse direkt auf dem Glassubstrat wird eine sehr gute optische Kopplung der Linse mit dem Glassubstrat erreicht, was zu einer verbesserten Kopplung mit angrenzenden Objektivgliedern führen kann.

Ferner könnten die Objektivglieder eine Blende aufweisen, die vorzugsweise in der optischen Achse vor der Linse angeordnet ist. Durch die Blende wird der Querschnitt von Strahlenbündeln begrenzt, die durch die Blende laufen und auf den Bildsensor treffen. Dadurch kann unter anderem die Schärfentiefe des Bildes bestimmt werden. Zur besonders kompakten Ausführung könnte die Blende als eine Beschichtung eines der Objektivglieder ausgeführt sein, wobei die Beschichtung lokal unterbrochen ist. Das Beschichtungsmaterial kann ein lichtundurchlässiges Material sein, etwa ein Metall, ein Metalloxid oder ein Pigmente oder Partikel aufweisendes Polymer. Die Blende kann beispielsweise durch physikalische Gasphasenabscheidung (physical vapour deposition, PVD) aufgedampft werden. Durch die Dimensionierung einer in der optischen Achse zentrierten Öffnung wird die Blendenfunktion realisiert. Die Blende könnte bevorzugt zwischen dem ersten Endplättchen und dem Glassubstrat angeordnet sein. Vorzugsweise kann die Blende eine Vorderseite und eine Rückseite bezüglich der optischen Achse aufweisen, wobei die Vorderseite und/oder die Rückseite einen Lichtreflexionsgrad von weniger als 10% aufweist. Dies ist besonders vorteilhaft für die Bildqualität, da Reflektionen an der Blendenvorderseite und/oder -rückseite zu unerwünschten Bildeffekten führen können. Der Lichtreflexionsgrad sei hier definiert als der Anteil einer reflektierten Lichtleistung von einer auf die Blende treffenden Lichtleistung.

Ferner könnte das Objektiv, abgesehen vom Bildgebungskanal, frei von einer eigenen Einfassung sein. Die Objektivglieder sind miteinander verbunden und werden zunächst ohne eine eigene umfangsseitige Einfassung bereitgestellt. Das Objektiv kann eine in radialer Richtung größtmögliche Größe annehmen und kann direkt in den Bildgebungskanal der distalen Schaftspitze eingesetzt werden. Durch die polygonale Form des Querschnitts kann eine formschlüssige, reibschlüssige und/oder stoffschlüssige Aufnahme und Einpassung in einem Bildgebungskanal des endoskopischen Instruments erfolgen. Die effektive optische Fläche eines Bildsensors wird vergrößert.

Erfindungsgemäß ist ein Bildsensor an dem Objektiv angeordnet und dazu ausgebildet, eine von dem Objektiv bereitgestellte optische Abbildung zu erfassen und in Form elektrischer Signale bereitzustellen. Während in einer möglichen anderen Variante das Objektiv über einen Bildleiter, d.h. ein geordnetes Bündel von Lichtleiterfasern zur optischen Bildübertragung, mit einem proximal angeordneten Bildsensor koppelbar wäre, könnte ein Bildsensor vorzugsweise direkt an dem Objektiv optisch angekoppelt werden. Es muss nun lediglich eine elektrische Leitung zwischen dem Bildsensor und einer Bildinformationen aufnehmenden Einrichtung angeordnet sein. Vorzugsweise wird der Bildsensor erst nach dem Herausschneiden des Objektivs aus dem Wafer-Level-Paket stoffschlüssig mit einer proximalen Seite des Objektivs verbunden. Dadurch können ggf. die lateralen Abmessungen des Objektivs kleiner ausgestaltet werden als die lateralen Abmessungen des Bildsensors.

In einer vorteilhaften Ausführungsform ist vorgesehen, dass zumindest ein distaler Abschnitt des Schafts, der das Objektiv aufweist, als Einmalgebrauchsartikel ausgebildet ist. Durch die besonders kostengünstige Herstellung des Objektivs kann eine aufwändige Reinigung und Wiederverwendung des distalen Abschnitts entfallen. Er könnte über eine geeignete Verbindung mit einem proximalen Abschnitt des endoskopischen Instruments verbunden sein und sich nach Verwendung lösen lassen, um es zu entsorgen.

Der Schaft des endoskopischen Instruments weist einen Bildgebungskanal und vorzugsweise zumindest zwei Arbeitskanäle auf, wobei das Objektiv formschlüssig, reibschlüssig und/oder stoffschlüssig in dem Bildgebungskanal eingesetzt ist. Durch die mindestens sechseckige Form des Querschnitts ist dieser einer Kreisform des Querschnitts des Bildgebungskanals näher als ein viereckiger und insbesondere quadratischer Querschnitt. Die sechs oder mehr Ecken können sich radial gut in die Wandung des Bildgebungskanals schmiegen und das Objektiv zentrieren, ohne größere Materialspannungen in dem Bildgebungskanal und in dem Objektiv hervorzurufen. Das reibschlüssige Einsetzen ist weiterhin mechanisch einfach zu realisieren.

Besonders bevorzugt ist das Objektiv fluiddicht mit einer distalen Öffnung des Bildgebungskanals verklebt und dichtet den Bildgebungskanal dadurch nach außen hin ab. Es ist folglich keine weitere Maßnahme notwendig, um den Bildgebungskanal für den Gebrauch abzudichten. In Kombination mit der kostengünstigen und sehr passgenauen Herstellung des Objektivs fallen insgesamt für den Bildgebungskanal eines endoskopischen Instruments nur sehr überschaubare Kosten an, die überdies die Ausführung als Einmalgebrauchsartikel unterstützen.

Erfindungsgemäß weist der Bildgebungskanal einen ersten distalen Bildgebungskanalabschnitt auf, in dem das Objektiv eingesetzt ist, und einen proximal vom ersten Bildgebungskanalabschnitt angeordneten zweiten Bildgebungskanalabschnitt, der einen größeren Innendurchmesser aufweist als der erste Bildgebungskanalabschnitt, wobei eine mit dem Objektiv stoffschlüssig verbundene Bildsensoreinheit im zweiten Bildgebungskanalabschnitt angeordnet ist. Die lateral größere Abmessungen aufweisende Bildsensoreinheit kann dadurch vom zweiten Bildgebungskanalabschnitt aufgenommen werden, während das Objektiv genau in den ersten Bildgebungskanalabschnitt passt.

Die Erfindung betrifft weiterhin ein Verfahren zum Herstellen endoskopischer Instrumente. Das Verfahren ist gekennzeichnet durch die Schritte des Bereitstellens mehrerer Wafer-Schichten eines optischen Materials, des Stapelns der Schichten zum Ausbilden eines Wafer-Pakets, und des Heraustrennens von Objektiven aus dem Wafer-Paket durch Schneiden des Wafer-Pakets mit zumindest drei Gruppen von parallelen Trennschnitten, deren Schnittrichtungen durch einen polygonalen und zumindest sechseckigen Querschnitt aller Objektive bestimmt werden. Außerdem folgt ein Schritt des stoffschlüssigen Verbindens der Objektive jeweils mit einer Bildsensoreinheit, wobei die Bildsensoreinheit laterale größere Abmessungen aufweist als das jeweilige Objektiv. Schließlich folgt ein Schritt des Einsetzens der mit der Bildsensoreinheit stoffschlüssig verbundenen Objektive jeweils in einen Bildgebungskanal einer distalen Spitze der herzustellenden endoskopischen Instrumente, wobei das Objektiv formschlüssig, reibschlüssig und/oder stoffschlüssig distalwärts in den Bildgebungskanal eingesetzt wird, wobei der Bildgebungskanal einen ersten distalen Bildgebungskanalabschnitt aufweist, in den das Objektiv eingesetzt wird, und einen proximal vom ersten Bildgebungskanalabschnitt angeordneten zweiten Bildgebungskanalabschnitt, der zur Aufnahme der Bildsensoreinheit einen größeren Innendurchmesser aufweist als der erste Bildgebungskanalabschnitt.

In einer vorteilhaften Ausführungsform werden beim Heraustrennen der Objektive drei Gruppen von parallelen Trennschnitten ausgeführt, wobei eine erste Schneidrichtung und eine zweite Schneidrichtung sowie die zweite Schneidrichtung und eine dritte Schneidrichtung jeweils einen Winkel von 60° einschließen. Damit können hexagonale Querschnitte realisiert werden.

Nachfolgend ist die Erfindung anhand von in den Zeichnungen dargestellten Ausführungsbeispielen näher erläutert. Es zeigen:
- Fig. 1 und 2: perspektivische Ansichten eines Objektivs mit einer daran angeordneten Bildsensoreinheit;
- Fig.3: eine perspektivische Ansicht des Objektivs ohne die Bildsensoreinheit;
- Fig.4: eine Explosionsdarstellung des Objektivs;
- Fig. 5: eine schematische Darstellung der wirksamen Bildsensorfläche auf dem Querschnitt des Objektivs;
- Fig. 6: eine Schnittdarstellung des Objektivs;
- Fig. 7 und 8: schematische Darstellungen des Heraustrennens von Objektiven aus einem Wafer-Paket mit hexagonalem Querschnitt (Fig. 7) und oktogonalem Querschnitt (Fig. 8);
- Fig. 9a bis 9d: verschiedene Darstellungen eines distalen Endes eines endoskopischen Instruments mit darin angeordnetem Objektiv;
- Fig. 10a bis 10c: verschiedene Darstellungen eines endoskopischen Instruments mit an der distalen Spitze angeordnetem Objektiv.

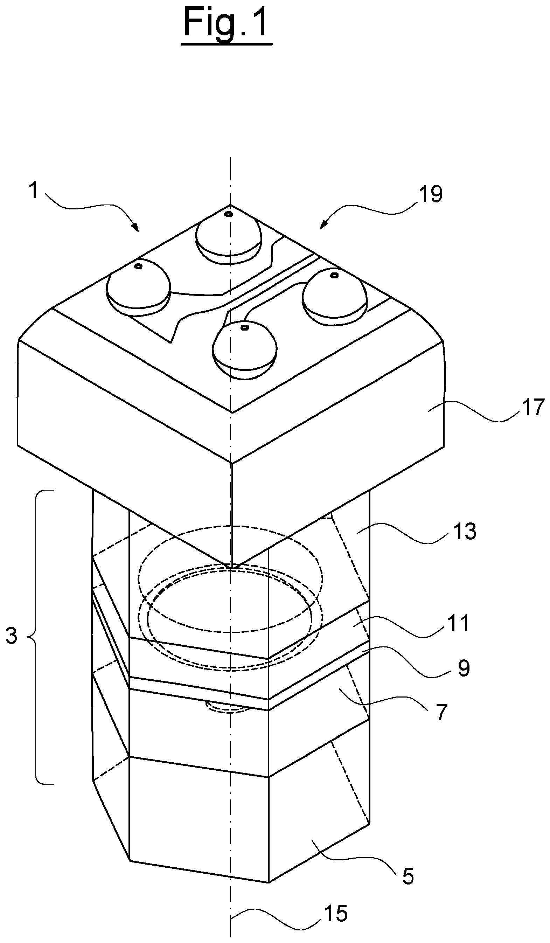

Fig. 1 zeigt ein Objektiv 1 mit einer daran angeordneten Bildsensoreinheit 17 für ein endoskopisches Instrument. Das Objektiv 1 mit einer daran angeordneten Bildsensoreinheit 17 weist eine aus einem mehrschichtigen Wafer-Paket herausgetrennte Anordnung 3 miteinander verbundener Objektivglieder auf. Die Objektivglieder sind vorzugsweise stoffschlüssig miteinander verbunden, z.B. miteinander verklebt. Diese sind als ein erstes Endplättchen 5, ein Glassubstrat 7, eine Linse 9, ein Distanzglied 11 und ein zweites Endplättchen 13 ausgeführt. Die Objektivglieder 5, 7, 9, 11 und 13 sind entlang einer optischen Achse 15 aufeinandergestapelt. Sie weisen in diesem Ausführungsbeispiel jeweils einen sechseckigen, gleichseitigen, d.h. hexagonalen, Querschnitt senkrecht zur optischen Achse 15 auf.

An dem zweiten Endplättchen 13 ist ferner eine Bildsensoreinheit 17 angeordnet, die dazu ausgebildet ist, ein von der Linse 9 erzeugtes Bild zu erfassen und an Signalanschlüssen 19 zur Verarbeitung bzw. Anzeige elektrisch bereitzustellen. Die lateralen Abmessungen der Bildsensoreinheit 17 sind größer als das Objektiv 1, jedoch kann die optisch wirksame Fläche des tatsächlichen Bildsensors (hier nicht sichtbar) quadratisch und etwas kleiner sein als der Querschnitt des Objektivs 1.

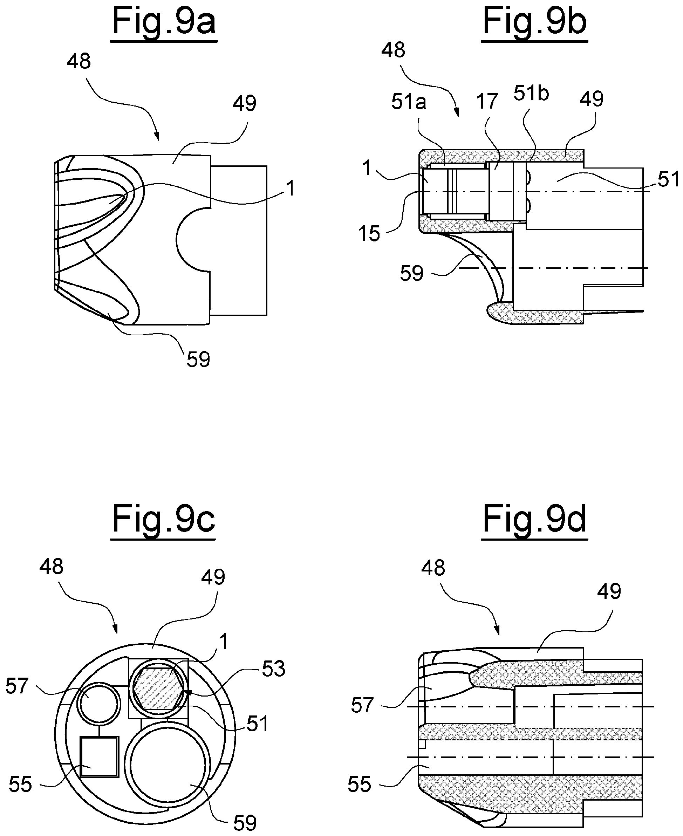

Der Vorteil der erfindungsgemäßen Gestaltung liegt in der besonders guten Ausnutzung des verfügbaren Querschnitts eines Bildgebungskanals eines endoskopischen Instruments (siehe Fig. 9a-d), der einen kreisrunden Querschnitt aufweist. Ein polygonaler Querschnitt, der mindestens sechseckig ist, kann sich vergleichsweise gut an eine kreisrunde Kontur anschmiegen. Eine aufwändige Nachbearbeitung zur Anpassung einer aus einem Wafer-Paket herausgetrennten Anordnung mit einem quadratischen Querschnitt kann entfallen. Zum Heraustrennen aus einem Wafer-Paket sind zwar zwei oder mehr Schnitte zusätzlich erforderlich, doch durch die fehlende Nachbearbeitung können insgesamt die Herstellkosten für ein Objektiv gegenüber dem Stand der Technik deutlich verringert werden. Eine eigene Einfassung zusätzlich zum Bildgebungskanal ist ferner nicht notwendig, da sich eine solche polygonale Kontur sehr gut reibschlüssig in einen Bildgebungskanal integrieren lässt.

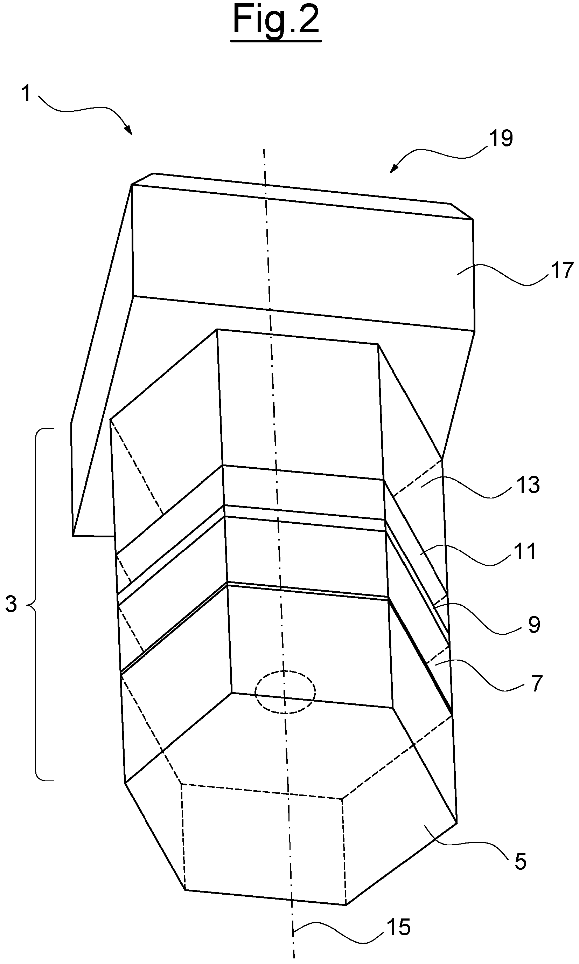

Fig. 2 zeigt das Objektiv 1 aus einer gegenüber Fig. 1 gekippten Perspektive. Die Bildsensoreinheit 17 ist an ihrer zu dem zweiten Endplättchen 13 gewandten Unterseite flach und liegt bündig auf dem zweiten Endplättchen 13 auf.

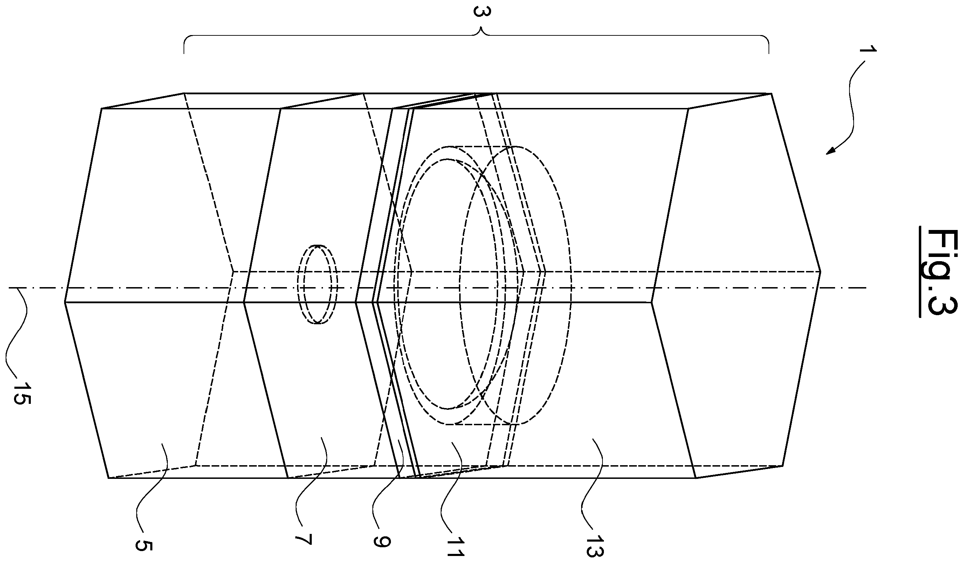

Fig. 3 zeigt das Objektiv aus derselben Perspektive wie in Fig. 1, jedoch ohne die Bildsensoreinheit 17. Das Objektiv 1 weist insgesamt eine längliche, zylindrische Form mit konstantem Querschnitt auf, wobei die Objektivglieder 5, 7, 9, 11 und 13 keine umfangsseitige Einfassung besitzen.

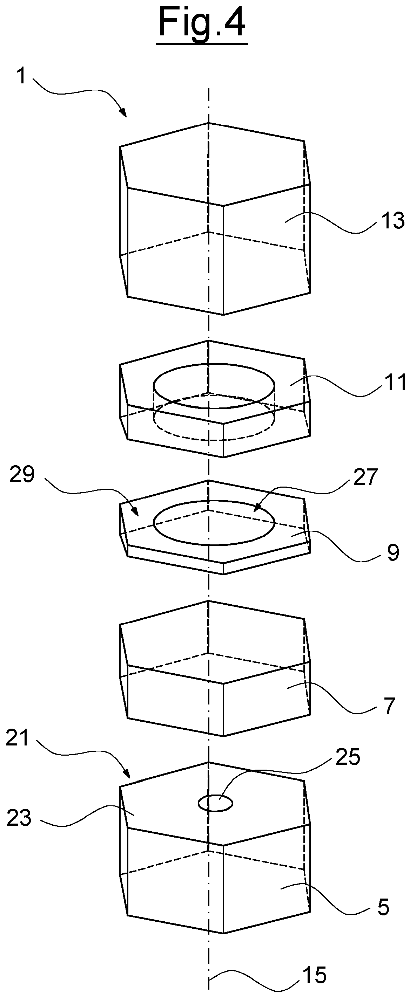

In Fig. 4 werden die Einzelteile des Objektivs 1 in einer Explosionsdarstellung gezeigt. Hier ist ersichtlich, dass das erste Endplättchen 5 und das zweite Endplättchen 13 jeweils den Querschnitt über ihre entlang der optischen Achse 15 gemessene Höhe vollständig und unterbrechungsfrei ausfüllen. An einer zu dem Glassubstrat 7 gewandten Oberfläche 21 des ersten Endplättchens 5, oder an einer dem Endplättchen 5 zugewandten Oberfläche des Glassubstrats 7, ist eine Blende in Form einer möglichst matten Blendenbeschichtung 23 angeordnet, die bis auf eine beispielhaft kreisrunde Blendenöffnung 25 die gesamte Oberfläche 21 abdeckt. Die Blendenbeschichtung 23 ist nicht lichtdurchlässig und könnte etwa Chrom, Chromoxid, Titan, Silizium, ein mit dunklen Partikeln versehenes Polymer oder ein anderes Material umfassen. Die Blendenbeschichtung 23 weist vorzugsweise einen Lichtreflexionsgrad von weniger als 10% nach oben und/oder unten auf. Die Blendenöffnung 25 weist einen auf die Linse 9 und die Bildsensoreinheit 17 angepassten Durchmesser auf.

Das Glassubstrat 7 könnte ein Trägersubstrat für die Linse 9 sein, welche aus einem UV-härtbaren Polymer auf dem Glassubstrat 7 aufgebaut sein kann. Die Linse 9 weist eine vorzugsweise asphärisch geformte Fläche 27 auf, die der Linse 9 eine zur gewünschten Lichtbündelung erforderliche Form verleiht. Zum Schutz der Linse ist das Distanzglied 11 auf einem flachen Rand 29 um die asphärische Fläche 27 herum angeordnet und erstreckt sich entlang der optischen Achse 15 weiter als die asphärische Fläche 27. Abschließend ist das zweite Endplättchen 13 vorgesehen, um das Objektiv 1 abzudecken.

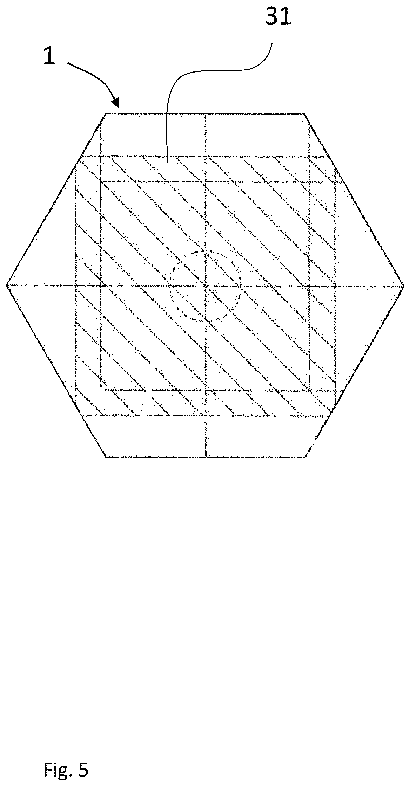

Fig. 5 zeigt beispielhaft eine Draufsicht auf das Objektiv 1 und eine optisch wirksame Fläche 31 eines Bildsensors der Bildsensoreinheit 17 (schraffiert). Diese Fläche 31 ist quadratisch und kann aufgrund der hier beispielhaft sechseckigen Form des Objektivs 1 ausreichend groß gewählt werden. Sie ist auf dem Querschnitt des Objektivs 1 zentriert und ragt bis an die in der Zeichnungsebene schräg angeordneten Seitenkanten des sechseckigen Querschnitts heran.

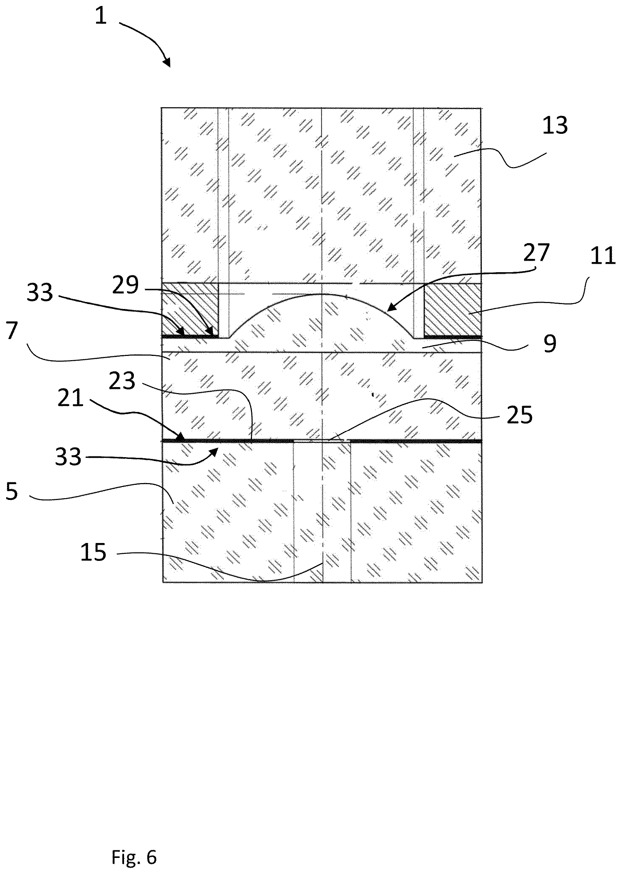

Fig. 6 zeigt eine Schnittdarstellung des Objektivs 1. Zwischen dem ersten Endplättchen 5 und dem Glassubstrat 7 ist zusätzlich zu der Blendenbeschichtung 23 außerdem eine Klebeschicht 33 vorgesehen, die das erste Endplättchen 5 mit dem Glassubstrat 7 verbindet. Auf dieselbe Weise ist auch der Rand 29 mit dem Distanzelement 11 verbunden. Das Distanzglied 11 und das zweite Endplättchen 13 sind stattdessen beispielhaft anodisch miteinander verbunden.

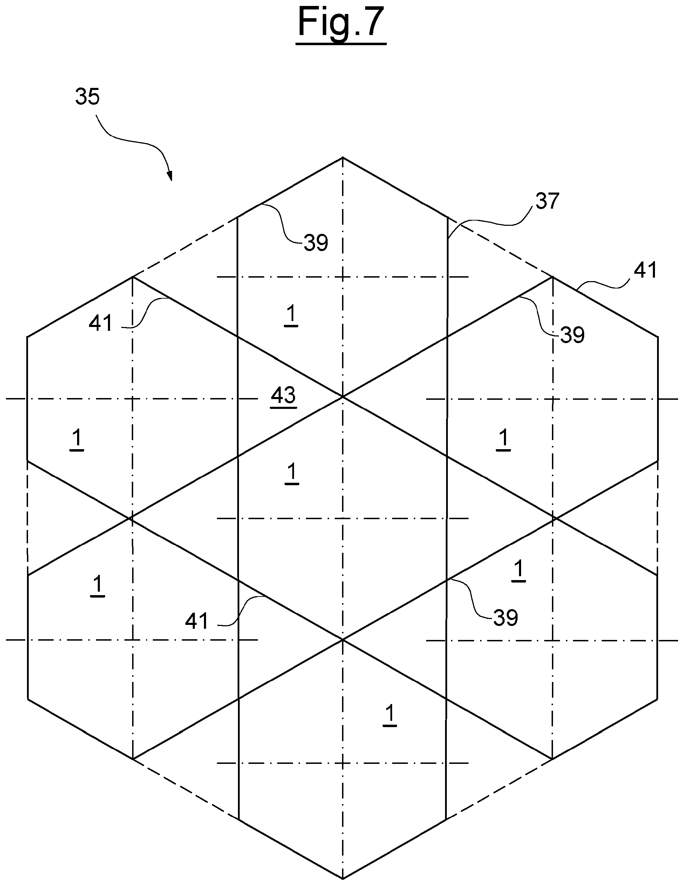

Fig. 7 zeigt einen Ausschnitt aus einem mehrschichtigen Wafer-Paket 35, wobei die einzelnen Schichten jeweils eine Vielzahl von ersten Endplättchen 5, Glassubstraten 7, Linsen 9, Distanzgliedern 11 und zweiten Endplättchen 13 bilden. Hier ist eine Reihe von Trennschnitten gezeigt, durch die einzelne Objektive 1 aus dem Wafer-Paket herausgetrennt werden können.

Eine Gruppe von ersten Trennschnitten 37 verläuft in der Zeichnungsebene in vertikaler Richtung. Mehrere erste Trennschnitte 37 sind parallel zueinander angeordnet, wobei ein Abstand der Mittellinien der ersten Trennschnitte 37 dem Abstand zweier gegenüberliegender Seiten bzw. Flächen des Objektivs 1 entspricht. Zur Ausformung mehrerer hexagonaler Objektive 1 sind weiterhin mehrere, parallel zueinander verlaufende zweite Trennschnitte 39 und mehrere, parallel zueinander verlaufende dritte Trennschnitte 41 vorgesehen. Die Abstände der zweiten Trennschnitte 39 zueinander sowie die Abstände der dritten Trennschnitte 41 zueinander sind mit den Abständen der ersten Trennschnitte 37 zueinander identisch. Die zweiten Trennschnitte 39 und die dritten Trennschnitte 41 sind jeweils um einen Winkel von 60° im Uhrzeigersinn bzw. gegen den Uhrzeigersinn zu den ersten Trennschnitten 37 angewinkelt.

Durch einen alternierenden, spaltenweisen Versatz parallel zu den ersten Trennschnitten 37 der herauszutrennenden Objektive 1 auf dem Wafer-Paket kann ein sehr geringer Verschnitt realisiert werden. Hierdurch fallen die gestrichelt gekennzeichneten dreieckigen Verschnittflächenabschnitte 43 an. Die gesamte Verschnittfläche entspricht über die genutzte Fläche des Wafer-Pakets 35 je Objektiv 1 einem Drittel der Querschnittsfläche eines Objektivs 1. Dieser Anteil kann bei anders geformten Polygonen hiervon abweichen.

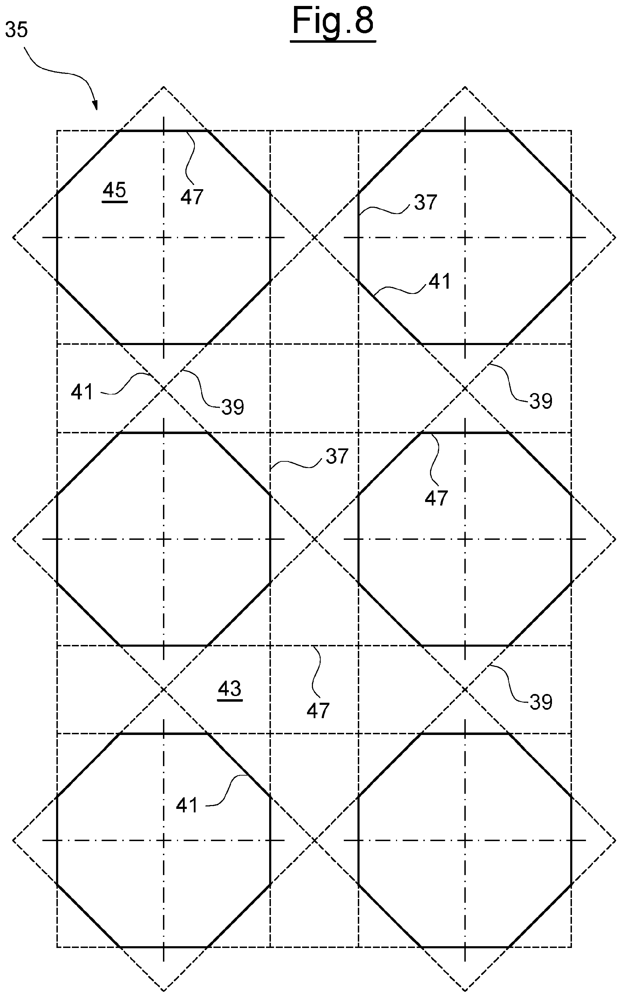

Zur Herstellung oktogonaler Objektive 45 zeigt Fig. 8 die möglichen durchzuführenden Trennschnitte. Hier sind neben ersten Trennschnitten 37, zweiten Trennschnitten 39 und dritten Trennschnitten 41 zudem vierte Trennschnitte 47 auszuführen. Die zweiten und dritten Trennschnitte sind jeweils um einen Winkel von 45° im Uhrzeigersinn bzw. gegen den Uhrzeigersinn zu den ersten Trennschnitten 37 angewinkelt. Die vierten Trennschnitte 47 sind ebenso parallel zueinander angeordnet und verlaufen senkrecht zu den ersten Trennschnitten 37.

Fig. 9a bis 9d zeigen eine distale Spitze 49 eines endoskopischen Instruments 48 in verschiedenen Ansichten. Fig. 9a ist eine Seitenansicht, Fig. 9b ein Seitenschnitt durch die optische Achse 15, Fig. 9c eine Draufsicht und Fig. 9d ein Seitenschnitt durch einen Arbeitskanal.

Die distale Spitze 49 weist einen Bildgebungskanal 51 auf, der an dem distalen Ende des endoskopischen Instruments 48 einen kreisrunden Querschnitt besitzt. Hier ist das Objektiv 1 aus der vorhergehenden Beschreibung reibschlüssig eingesetzt, sodass sich Ecken 53 des Querschnitts des Objektivs 1 bündig in den Bildgebungskanal 51 drücken. Zusätzlich kann das Objektiv 1 eingeklebt sein, um den Bildgebungskanal 51 fluiddicht nach außen hin abzudichten. Der Bildgebungskanal 51 weist einen ersten distalen Bildgebungskanalabschnitt 51a auf, in dem das Objektiv 1 eingesetzt ist, und einen proximal vom ersten Bildgebungskanalabschnitt 51a angeordneten zweiten Bildgebungskanalabschnitt 51b, der einen größeren Innendurchmesser aufweist als der erste Bildgebungskanalabschnitt 51a. Die mit dem Objektiv 1 stoffschlüssig proximalseitig verbundene Bildsensoreinheit 17 ist dabei im zweiten Bildgebungskanalabschnitt 51b angeordnet. Die lateralen Abmessungen der im zweiten Bildgebungskanalabschnitt 51b angeordneten Bildsensoreinheit 17 sind etwas größer als die lateralen Abmessungen des Objektivs 1, das passgenau in den ersten Bildgebungskanalabschnitt 51a eingepasst ist. Das Objektiv 1 mit der Bildsensoreinheit 17 wird vorzugsweise als vormontierte Einheit von proximal, also distalwärts, in die Spitze 49 eingepasst.

Wie in Fig. 9c zu erkennen, weist die distale Spitze 49 weiterhin eine Beleuchtungseinheit 55 auf, die den vor der Spitze 49 liegenden, zu beobachtenden Bereich beleuchtet, um eine Bildgebung davon zu ermöglichen. Beispielhaft kann die Beleuchtungseinheit 55 in Form eines LED-Elements mit beispielhaft rechteckigem bzw. quadratischem Querschnitt realisiert werden.

Neben dem Bildgebungskanal 51 und der Beleuchtungseinheit 55 enden ein erster Arbeitskanal 57 und ein zweiter Arbeitskanal 59 in der distalen Spitze 49. Der erste Arbeitskanal 57 weist exemplarisch eine deutlich geringere Querschnittsfläche auf als der zweite Arbeitskanal 59 und könnte beispielhaft zum Durchschieben eines Laserlichtleiters verwendet werden. Der zweite Arbeitskanal 59 kann indes zum bedarfsweisen Leiten von Spülflüssigkeit und/oder für ein endoskopisches Werkzeug eingesetzt werden.

Fig. 9b zeigt einen Schnitt durch die optische Achse 15, d.h. den Bildgebungskanal 51, und stellt das Objektiv 1 dar, an dessen proximalen Ende die Bildsensoreinheit 17 angeordnet ist. Diese wird mit einer hier nicht gezeigten elektrischen Leitung verbunden, welche sich in proximaler Richtung erstreckt. Der Vollständigkeit halber wird in Fig. 9d ein Schnitt durch den ersten Arbeitskanal 57 gezeigt.

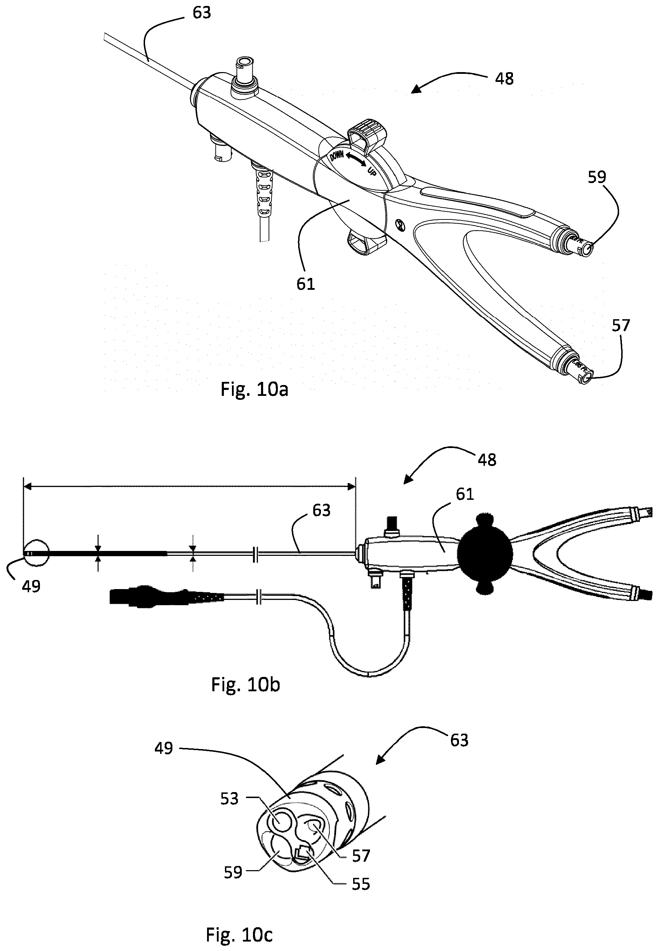

Fig. 10a zeigt einen proximalen Teil des endoskopischen Instruments 48 mit einer manuell handhabbaren Handhabungseinrichtung 61, von der sich distalwärts ein starrer rohrförmiger dünner Schaft 63 erstreckt. Der Schaft 63 ist vorzugsweise an einem distalen Endabschnitt abwinkelbar bzw. krümmbar, um die distale Spitze 49 des Schafts 63 gewünscht ausrichten zu können. In Fig. 10b ist das endoskopische Instrument 48 als Einwegartikel als Ganzes gezeigt, wobei der Schaft 63 gekürzt dargestellt ist. Der Schaft 63 ist in Wirklichkeit um ein Vielfaches länger als die Handhabungseinrichtung 61. Fig. 10c zeigt die distale Spitze 49 des Schafts des endoskopischen Instruments 48, wie sie in Fig. 9a-d genauer dargestellt ist.

### Bezugszeichenliste

- 1: Objektiv (hexagonal)
- 3: Anordnung
- 5: erstes Endplättchen
- 7: Glassubstrat
- 9: Linse
- 11: Distanzglied
- 13: zweites Endplättchen
- 15: optische Achse
- 17: Bildsensoreinheit
- 19: Signalanschlüsse
- 21: Oberfläche des ersten Endplättchens
- 23: Blendenbeschichtung / Blende
- 25: Blendenöffnung
- 27: asphärische Fläche
- 29: Rand
- 31: wirksame Fläche
- 33: Klebeschicht
- 35: Wafer-Paket
- 37: erster Trennschnitt
- 39: zweiter Trennschnitt
- 41: dritter Trennschnitt
- 43: Verschnittflächenabschnitt
- 45: Objektiv (oktagonal)
- 47: vierter Trennschnitt
- 48: endoskopisches Instrument
- 49: distale Spitze
- 51: Bildgebungskanal
- 51a: erster Bildgebungskanalabschnitt
- 51b: zweiter Bildgebungskanalabschnitt
- 53: Ecke
- 55: Beleuchtungseinheit
- 57: erster Arbeitskanal
- 59: zweiter Arbeitskanal
- 61: Handhabungseinrichtung
- 63: Schaft

## Patentansprüche

1. Endoskopisches Instrument (48), aufweisend einen rohrförmigen Schaft (63), an dessen distaler Spitze (49) ein Objektiv (1, 45) angeordnet ist, das eine aus einem mehrschichtigen Wafer-Paket herausgetrennte Anordnung (3) miteinander verbundener Objektivglieder (5, 7, 9, 11, 13) aufweist, die jeweils vorgegebene optische Eigenschaften aufweisen und entlang einer optischen Achse (15) aufeinander folgen, wobei die Anordnung (3) einen senkrecht zu der optischen Achse polygonalen und zumindest sechseckigen Querschnitt aufweist, wobei die distale Spitze (49) einen Bildgebungskanal (51) aufweist, wobei der Bildgebungskanal (51) an einem distalen Ende des endoskopischen Instruments (48) einen kreisrunden Querschnitt besitzt, wobei das Objektiv (1, 45) reibschlüssig in dem Bildgebungskanal (51) eingesetzt ist, sodass sich Ecken (53) des Querschnitts des Objektivs (1) bündig in den Bildgebungskanal (51) drücken, wobei das Objektiv (1, 45) eingeklebt ist, um den Bildgebungskanal (51) fluiddicht nach außen hin abzudichten, wobei der Bildgebungskanal (51) einen ersten distalen Bildgebungskanalabschnitt (51a) aufweist, in dem das Objektiv (1, 45) eingesetzt ist, und einen proximal vom ersten Bildgebungskanalabschnitt (51a) angeordneten zweiten Bildgebungskanalabschnitt (51b), der einen größeren Innendurchmesser aufweist als der erste Bildgebungskanalabschnitt (51a), wobei eine mit dem Objektiv (1, 45) stoffschlüssig verbundene Bildsensoreinheit (17) im zweiten Bildgebungskanalabschnitt (51b) angeordnet ist, wobei die Bildsensoreinheit (17) größere laterale Abmessungen aufweist als das Objektiv (1, 45).

2. Endoskopisches Instrument (48) nach Anspruch 1, wobei der Querschnitt des Objektivs (1, 45) gleichseitig polygonal ist und bevorzugt hexagonal oder oktogonal ist.

3. Endoskopisches Instrument (48) nach Anspruch 1 oder 2, wobei die Objektivglieder (5, 7, 9, 11, 13) ein erstes Endplättchen (5) und ein zweites Endplättchen (13) aufweisen, die die Anordnung (3) aneinander entgegengesetzten stirnseitigen Enden begrenzen.

4. Endoskopisches Instrument (48) nach einem der vorhergehenden Ansprüche, wobei die Objektivglieder (5, 7, 9, 11, 13) zumindest eine Linse (9) aufweisen.

5. Endoskopisches Instrument (48) nach Anspruch 4, wobei die Linse (9) eine asphärisch geformte Fläche (27) aufweist, die von einem flachen Rand (29) umgeben ist, wobei ein Distanzglied (11) auf dem Rand (29) angeordnet ist und sich entlang der optischen Achse (15) über die asphärische Fläche (27) hinaus erstreckt.

6. Endoskopisches Instrument (48) nach Anspruch 4 oder 5, wobei die Linse (9) auf einer ebenen und durchgehenden Fläche eines Glassubstrats (7) angeordnet ist.

7. Endoskopisches Instrument (48) nach einem der vorhergehenden Ansprüche, wobei die Objektivglieder (5, 7, 9, 11, 13) eine Blende (23) aufweisen.

8. Endoskopisches Instrument (48) nach Anspruch 7, wobei die Blende (23) in der optischen Achse (15) vor der Linse (9) angeordnet ist.

9. Endoskopisches Instrument (48) nach Anspruch 7 oder 8, wobei die Blende (23) eine Vorderseite und eine Rückseite bezüglich der optischen Achse (15) aufweist, wobei die Vorderseite und/oder die Rückseite einen Lichtreflexionsgrad von weniger als 10% aufweist.

10. Endoskopisches Instrument (48) nach einem der vorhergehenden Ansprüche, wobei die Bildsensoreinheit (17) dazu ausgebildet ist, eine von dem Objektiv (1, 45) bereitgestellte optische Abbildung zu erfassen und in Form elektrischer Signale bereitzustellen.

11. Endoskopisches Instrument (48) nach einem der vorhergehenden Ansprüche, wobei zumindest ein distaler Abschnitt des Schafts (63), der das Objektiv (1, 45) aufweist, als Einmalgebrauchsartikel ausgebildet ist.

12. Endoskopisches Instrument (48) nach einem der vorhergehenden Ansprüche, wobei das Objektiv (1, 45) fluiddicht mit einer distalen Öffnung des Bildgebungskanals (51) verklebt ist und den Bildgebungskanal (51) dadurch nach außen hin abdichtet.

13. Verfahren zum Herstellen endoskopischer Instrumente (48), **gekennzeichnet durch** die Schritte:
- Bereitstellen mehrerer Wafer-Schichten eines optischen Materials,
- Stapeln der Schichten zum Ausbilden eines Wafer-Pakets, und
- Heraustrennen von Objektiven (1, 45) aus dem Wafer-Paket durch Schneiden des Wafer-Pakets mit zumindest drei Gruppen von parallelen Trennschnitten (37, 39, 41, 47), deren Schnittrichtungen durch einen polygonalen und zumindest sechseckigen Querschnitt aller Objektive (1, 45) bestimmt werden
- stoffschlüssiges Verbinden der Objektive (1, 45) jeweils mit einer Bildsensoreinheit (17), wobei die Bildsensoreinheit (17) größere laterale Abmessungen aufweist als das jeweilige Objektiv (1, 45),
- Einsetzen der mit der Bildsensoreinheit (17) stoffschlüssig verbundenen Objektive (1, 45) jeweils in einen Bildgebungskanal (51) mit kreisrundem Querschnitt einer distalen Spitze (49) der herzustellenden endoskopischen Instrumente (48), wobei das Objektiv (1, 45) reibschlüssig distalwärts in den Bildgebungskanal (51) derart eingesetzt wird, dass sich Ecken (53) des Querschnitts des Objektivs (1) bündig in den Bildgebungskanal (51) drücken, wobei das Objektiv (1, 45) eingeklebt wird, um den Bildgebungskanal (51) fluiddicht nach außen hin abzudichten, wobei der Bildgebungskanal (51) einen ersten distalen Bildgebungskanalabschnitt (51a) aufweist, in den das Objektiv (1, 45) eingesetzt wird, und einen proximal vom ersten Bildgebungskanalabschnitt (51a) angeordneten zweiten Bildgebungskanalabschnitt (51b), der zur Aufnahme der Bildsensoreinheit (17) einen größeren Innendurchmesser aufweist als der erste Bildgebungskanalabschnitt (51a).

14. Verfahren nach Anspruch 13, wobei beim Heraustrennen der Objektive (1, 45) mindestens drei Gruppen von parallelen Trennschnitten (37, 39, 41, 47) ausgeführt werden, wobei eine erste Schneidrichtung und eine zweite Schneidrichtung sowie die zweite Schneidrichtung und eine dritte Schneidrichtung jeweils einen Winkel von 60° einschließen.

## Claims

1. An endoscopic instrument (48), having a tubular shaft (63), at the distal tip (49) of which there is arranged an objective (1, 45) that has an assembly (3), said assembly being separated from a multilayer wafer packet, of interconnected objective elements (5, 7, 9, 11, 13), said objective elements each having specified optical properties and following one another along an optical axis (15), wherein the assembly (3) has a cross-section which is polygonal perpendicularly to the optical axis and which has at least six corners, wherein the distal tip (49) has an imaging channel (51), wherein the imaging channel (51) has a circular cross-section at a distal end of the endoscopic instrument (48), wherein the objective (1, 45) is inserted in the imaging channel (51) in a frictionally engaged manner, so that corners (53) of the cross-section of the objective (1) press flush into the imaging channel (51), wherein the objective (1, 45) is glued in, so as to seal the imaging channel (51) fluid-tightly outwardly, wherein the imaging channel (51) has a first distal imaging channel portion (51a), in which the objective (1, 45) is inserted, and a second imaging channel portion (51b), which is arranged proximally of the first imaging channel portion (51a) and has a greater inner diameter than the first imaging channel portion (51a), wherein an image sensor unit (17) which is integrally bonded to the objective (1, 45) is arranged in the second imaging channel portion (51b), wherein the image sensor unit (17) has greater lateral dimensions than the objective (1, 45).

2. The endoscopic instrument (48) according to claim 1, wherein the cross-section of the objective (1, 45) is equilaterally polygonal and is preferably hexagonal or octagonal.

3. The endoscopic instrument (48) according to claim 1 or 2, wherein the objective elements (5, 7, 9, 11, 13) have a first end plate (5) and a second end plate (13), which delimit the assembly (3) at end faces opposite one another.

4. The endoscopic instrument (48) according to one of the preceding claims, wherein the objective elements (5, 7, 9, 11, 13) have at least one lens (9).

5. The endoscopic instrument (48) according to claim 4, wherein the lens (9) has an aspherically shaped surface (27), which is surrounded by a flat edge (29), wherein a spacer (11) is arranged on the edge (29) and extends along the optical axis (15) beyond the aspherical surface (27).

6. The endoscopic instrument (48) according to claim 4 or 5, wherein the lens (9) is arranged on a flat and continuous surface of a glass substrate (7).

7. The endoscopic instrument (48) according to one of the preceding claims, wherein the objective elements (5, 7, 9, 11, 13) have a diaphragm (23).

8. The endoscopic instrument (48) according to claim 7, wherein the diaphragm (23) is arranged in front of the lens (9) in the optical axis (15).

9. The endoscopic instrument (48) according to claim 7 or 8, wherein the diaphragm (23) has a front side and a rear side with respect to the optical axis (15), wherein the front side and/or the rear side have/has a light reflectance value of less than 10%.

10. The endoscopic instrument (48) according to one of the preceding claims, wherein the image sensor unit (17) is configured to detect an optical imaging provided by the objective (1, 45) and to provide the optical imaging in the form of electrical signals.

11. The endoscopic instrument (48) according to one of the preceding claims, wherein at least a distal portion of the shaft (63), which comprises the objective (1, 45), is configured as a disposable article.

12. The endoscopic instrument (48) according to one of the preceding claims, wherein the objective (1, 45) is fluid-tightly bonded to a distal opening of the imaging channel (51) and thus seals the imaging channel (51) outwards.

13. A method for producing endoscopic instruments (48), **characterized by** the steps of:
- providing a plurality of wafer layers of an optical material,
- stacking the layers to form a wafer packet, and
- separating objectives (1, 45) from the wafer packet by cutting the wafer packet with at least three groups of parallel separating cuts (37, 39, 41, 47), the cut directions of which are determined by a cross-section of all objectives (1, 45) that is polygonal and has at least six corners,
- integrally bonding each of the objectives (1, 45) to an image sensor unit (17), wherein the image sensor unit (17) has larger lateral dimensions than the particular objective (1, 45),
- inserting each of the objectives (1, 45) integrally bonded to the image sensor unit (17) into an imaging channel (51) with circular cross-section of a distal tip (49) of the endoscopic instruments (48) to be produced, wherein the objective (1, 45) is inserted in a frictionally engaged manner distally into the imaging channel (51) in such a way that corners (53) of the cross-section of the objective (1) press flush into the imaging channel (51), wherein the objective (1, 45) is glued in, so as to seal the imaging channel (51) fluid-tightly outwards, wherein the imaging channel (51) has a first distal imaging channel portion (51a), into which the objective (1, 45) is inserted, and a second imaging channel portion (51b), which is arranged proximally of the first imaging channel portion (51a) and, to receive the image sensor unit (17), has a greater inner diameter than the first imaging channel portion (51a).

14. The method according to claim 13, wherein, when separating the objectives (1, 45), at least three groups of parallel separating cuts (37, 39, 41, 47) are made, wherein a first cutting direction and a second cutting direction, and also the second cutting direction and a third cutting direction, in each case form an angle of 60°.

## Revendications

1. Instrument endoscopique (48) présentant une tige tubulaire (63) sur la pointe distale (49) de laquelle est disposé un objectif (1, 45) qui présente un agencement (3) d'éléments d'objectif (5, 7, 9, 11, 13) reliés entre eux, séparé d'un paquet de plaquettes multi-couches, qui présentent respectivement des propriétés optiques prédéfinies et se suivent le long d'un axe optique (15), dans lequel l'agencement (3) présente une section polygonale et au moins hexagonale perpendiculaire à l'axe optique, dans lequel la pointe distale (49) présente un canal d'imagerie (51), dans lequel le canal d'imagerie (51) possède une section ronde à une extrémité distale de l'instrument endoscopique (48), dans lequel l'objectif (1, 45) est inséré dans le canal d'imagerie (51) par friction, de sorte que des coins (53) de la section de l'objectif (1) appuient dans le canal d'imagerie (51) en affleurant, dans lequel l'objectif (1, 45) est collé pour étanchéifier le canal d'imagerie (51) vers l'extérieur contre les fluides, dans lequel le canal d'imagerie (51) présente un premier tronçon de canal d'imagerie (51a) distal dans lequel l'objectif (1, 45) est inséré, et un deuxième tronçon de canal d'imagerie (51b) proximal par rapport au premier tronçon de canal d'imagerie (51a) qui présente un diamètre interne plus grand que le premier tronçon de canal d'imagerie (51a), dans lequel une unité de capteur d'image (17) reliée par adhérence de matière avec l'objectif (1, 45) est disposée dans le deuxième tronçon de canal d'imagerie (51b), dans lequel l'unité de capteur d'image (17) présente des dimensions plus grandes que l'objectif (1, 45).

2. Instrument endoscopique (48) selon la revendication 1, dans lequel la section de l'objectif (1, 45) est polygonale équilatérale et de préférence hexagonale ou octogonale.

3. Instrument endoscopique (48) selon la revendication 1 ou 2, dans lequel les éléments d'objectif (5, 7, 9, 11, 13) présentent une première plaquette d'extrémité (5) et une deuxième plaquette d'extrémité (13) qui délimitent l'agencement (3) à des extrémités frontales opposées l'une l'autre.

4. Instrument endoscopique (48) selon l'une des revendications précédentes, dans lequel les éléments d'objectif (5, 7, 9, 11, 13) présentent au moins une lentille (9).

5. Instrument endoscopique (48) selon la revendication 4, dans lequel la lentille (9) présente une surface asphérique (27) qui est entourée d'un bord plat (29), dans lequel un élément distanceur (11) est disposé sur le bord (29) et s'étend le long de l'axe optique (15) au-delà de la surface asphérique (27).

6. Instrument endoscopique (48) selon la revendication 4 ou 5, dans lequel la lentille (9) est disposée sur une surface plane et traversante d'un substrat de verre (7).

7. Instrument endoscopique (48) selon l'une des revendications précédentes, dans lequel les éléments d'objectif (5, 7, 9, 11, 13) présentent un obturateur (23).

8. Instrument endoscopique (48) selon la revendication 7, dans lequel l'obturateur (23) est disposé devant la lentille (9) dans l'axe optique (15).

9. Instrument endoscopique (48) selon la revendication 7 ou 8, dans lequel l'obturateur (23) présente une face avant et une face arrière par rapport à l'axe optique (15), dans lequel la face avant et/ou la face arrière présente(nt) un degré de réflexion de la lumière de moins de 10 %.

10. Instrument endoscopique (48) selon l'une des revendications précédentes, dans lequel l'unité de capteur d'image (17) est conçue pour détecter une image optique fournie par l'objectif (1, 45) et la fournir sous forme de signaux électriques.

11. Instrument endoscopique (48) selon l'une des revendications précédentes, dans lequel au moins un tronçon distal de la tige (63) qui présente l'objectif (1, 45) est conçu en tant qu'article à usage unique.

12. Instrument endoscopique (48) selon l'une des revendications précédentes, dans lequel l'objectif (1, 45) est collé en étanchéité aux fluides à une ouverture distale du canal d'imagerie (51) et étanchéifie ainsi le canal d'imagerie (51) vers l'extérieur.

13. Procédé de fabrication d'instruments endoscopiques (48), **caractérisé par** les étapes:
- de fourniture de plusieurs couches de plaquettes d'un matériau optique,
- d'empilement des couches pour former un paquet de plaquettes, et
- de séparation des objectifs (1, 45) hors du paquet de plaquettes par découpe du paquet de plaquettes avec au moins trois groupes de coupes de séparation parallèles (37, 39, 41, 47) dont les sens de coupe sont déterminés par une section polygonale et au moins hexagonale de tous les objectifs (1, 45)
- de liaison par adhérence de matière des objectifs (1, 45) respectivement avec une unité de capteur d'image (17), dans lequel l'unité de capteur d'image (17) présente des dimensions plus grandes que l'objectif (1, 45) respectif,
- d'insertion des objectifs (1, 45) reliés par adhérence de matière à l'unité de capteur d'image (17) respectivement dans un canal d'imagerie (51) de section ronde d'une pointe distale (49) des instruments endoscopiques (48) à fabriquer, dans lequel l'objectif (1, 45) est ainsi inséré dans le canal d'imagerie (51) vers la partie distale par friction de telle sorte que des coins (53) de la section de l'objectif (1) appuient dans le canal d'imagerie (51) en affleurant, dans lequel l'objectif (1, 45) est collé pour étanchéifier le canal d'imagerie (51) contre les fluides vers l'extérieur, dans lequel le canal d'imagerie (51) présente un premier tronçon de canal d'imagerie (51a) distal dans lequel l'objectif (1, 45) est inséré, et un deuxième tronçon de canal d'imagerie (51b) proximal par rapport au premier tronçon de canal d'imagerie (51a) qui présente un diamètre interne plus grand que le premier tronçon de canal d'imagerie (51a) pour recevoir l'unité de capteur d'image (17).

14. Procédé selon la revalorisation 13, dans lequel lors de la séparation des objectifs (1, 45) au moins trois groupes de coupes de séparation parallèles (37, 39, 41, 47) sont réalisés, dans lequel un premier sens de coupe et un deuxième sens de coupe ainsi que le deuxième sens de coupe et un troisième sens de coupe incluent respectivement un angle de 60°.
